# FASCICULE DE BREVET EUROPEEN

(11) **EP 3 387 408 B1**
(45) Date de publication et mention de la délivrance du brevet: **16.10.2019**
(21) Numéro de dépôt: 16808600.7
(22) Date de dépôt: 05.12.2016
(51) Int. Cl.: G01M 3/20, G01N 33/00, G06F 3/01, G01N 1/24

(54) **DISPOSITIF DE DÉTECTION DE FUITES ET MODULE DE DÉTECTION DE FUITES**
LECKERKENNUNGSVORRICHTUNG UND LECKERKENNUNGSMODUL
LEAK-DETECTING DEVICE AND LEAK-DETECTING MODULE

(30) Priorité: 07.12.2015 FR 1561927
(43) Date de publication de la demande: 17.10.2018
(73) Titulaire: Pfeiffer Vacuum, 74000 Annecy (FR)
(72) Inventeur: JOURDAN, Pascal, 74330 Poisy (FR); DUCIMETIERE, Laurent, 74320 Sevrier (FR)
(74) Mandataire: Croonenbroek, Thomas Jakob
(86) Numéro de dépôt international: PCT/EP2016/079769
(87) Numéro de publication internationale: WO 2017/097711

(56) Documents cités:
- WO-A2-00/27478
- CN-A- 103 040 168
- DE-A1- 2 801 345
- FR-A1- 2 992 723
- JP-A- 2003 018 443
- US-A1- 2008 314 774
- US-A1- 2011 247 399
- US-A1- 2013 104 283

## Description

La présente invention concerne un dispositif de détection de fuites et un module de détection de fuites comprenant ledit dispositif pour contrôler l'étanchéité d'un objet à tester par gaz traceur.

Une méthode connue pour contrôler l'étanchéité d'un objet consiste à réaliser un test dit « en reniflage » ou « en aspersion » de gaz traceur. Cette méthode fait appel à la détection du passage du gaz traceur à travers les éventuelles fuites de l'objet à tester.

En mode reniflage, on recherche à l'aide d'un détecteur de fuites relié à une sonde de reniflage la présence éventuelle du gaz traceur autour d'un objet à tester rempli avec le gaz traceur généralement pressurisé.

En mode d'aspersion, on asperge avec un pistolet d'aspersion l'objet à tester de gaz traceur, le volume intérieur de l'objet à tester étant relié à un détecteur de fuites.

La recherche de fuites est réalisée en déplaçant l'extrémité de la sonde de reniflage ou le pistolet d'aspersion autour de l'objet à tester, notamment au niveau des zones susceptibles de présenter des faiblesses d'étanchéité, telles qu'autour des joints d'étanchéité.

Cependant, certaines zones peuvent être difficiles d'accès, notamment lorsque les objets à tester présentent des formes complexes et irrégulières ou dans le cas de zones à tester non visibles par l'utilisateur, comme des zones sombres ou des zones arrières. Ces difficultés d'accès peuvent être accentuées par la forme généralement étroite, longue et rigide de l'extrémité du pistolet d'aspersion et de la sonde de reniflage. La manipulation par l'utilisateur n'est donc pas aisée notamment en aveugle.

Certaines installations à tester présentent en plus de grandes dimensions, ce qui peut compliquer la recherche de fuites. En effet, l'encombrement de l'installation peut contraindre l'utilisateur à adopter des postures instables, le forçant à s'aider de la structure de l'installation pour atteindre des zones difficiles d'accès. Les mains de l'utilisateur peuvent alors être sollicitées pour permettre son maintien. Toutefois, l'utilisateur peut avoir besoin d'avoir de ses mains libres pour la manipulation d'outils, par exemple pour démonter un capot de protection ou pour éclairer la zone de recherche. Egalement, au cours de la recherche, l'utilisateur peut se retrouver éloigné du détecteur de fuites et avoir besoin de manipuler une télécommande lui permettant de piloter un détecteur de fuites à distance.

Par ailleurs, la manipulation du pistolet d'aspersion ou de la sonde de reniflage peut provoquer des douleurs aux mains de l'opérateur chargé de tester l'étanchéité d'objets de manière répétée, continuelle et quotidienne.

Le document CN103040168 propose d'insérer dans un gant un détecteur d'alcoolémie pour mesurer la quantité d'alcool passant à travers un trou dans le gant. Le document WO00/27478 décrit un gant à double paroi qui possède des moyen intégrés dans le gant pour détecter une fuite dans la paroi extérieure. Le document FR2992723 décrit un gant portant des moyens de détection de fuite par ultrason. Le document US2011/0247399 décrit une soufflette portable en forme de pistolet pour asperger un gaz traceur.

Un des buts de la présente invention est donc de proposer un dispositif de détection de fuites et un module de détection qui résolvent au moins en partie les inconvénients précités, notamment en proposant un dispositif de détection de fuites plus ergonomique et plus facile à manipuler.

A cet effet, l'invention a pour objet un dispositif de détection de fuites configuré pour contrôler l'étanchéité d'un objet à tester par gaz traceur, ledit dispositif de détection de fuites selon la revendication 1 comprenant une sonde de reniflage destinée à être reliée à un détecteur de fuites et un dispositif de détection de fuites selon la revendication 2 comprenant une soufflette d'aspersion destinée à être reliée à une source de gaz comportant un gant auquel la la soufflette d'aspersion est fixée.

La sonde de reniflage et/ou la soufflette d'aspersion portée par le gant permet ainsi à l'utilisateur une manipulation plus intuitive et plus ergonomique. Il peut ainsi accéder plus facilement aux zones de l'objet à tester peu visibles, telles que des zones arrières ou sombres, avec la dextérité que lui procure l'adresse de sa main et avec un repérage spatial facilité par le contact manuel de l'objet à tester. De plus, le port de la sonde de reniflage et/ou de la soufflette d'aspersion par le gant n'immobilise plus la main de l'utilisateur qui peut l'employer à d'autres fins. On évite en outre les douleurs aux mains liées à une prise prolongée des renifleurs/pistolets de l'art antérieur.

Selon une ou plusieurs caractéristiques du dispositif de détection de fuites, prise seule ou en combinaison :
- la canalisation de circulation de gaz est flexible,
- la canalisation de circulation de gaz traverse le gant en étant intégrée dans le gant,
- la canalisation de circulation de gaz s'étend le long d'une partie du gant destinée à couvrir un doigt de l'utilisateur, en particulier l'index, et débouche au niveau de l'extrémité de ladite partie du gant correspondant au bout du doigt,
- le dispositif de détection de fuites comporte un organe de contrôle porté par le gant et configuré pour communiquer avec une unité de contrôle du détecteur de fuites,
- l'organe de contrôle comporte au moins un moyen d'activation configuré pour commander le lancement d'une mesure de la concentration en gaz traceur et/ou d'une réinitialisation du bruit de fond du détecteur de fuites,
- le dispositif de détection de fuites comporte un dispositif d'affichage porté par le gant, configuré pour afficher un signal de mesure du détecteur de fuites et/ou pour donner une information de l'état du détecteur de fuites,
- le dispositif d'affichage comporte un écran configuré pour afficher une évolution de la concentration en gaz traceur au cours du temps mesuré par le détecteur de fuites,
- l'organe de contrôle et/ou le dispositif d'affichage comportent des moyens de communication sans fils pour communiquer avec une unité de contrôle du détecteur de fuites,
- le dispositif de détection de fuites comporte un dispositif d'éclairage porté par le gant,
- le dispositif de détection de fuites comporte une caméra portée par le gant et un système de vision déporté configuré pour afficher les images prises par la caméra,
- le système de vision déporté est configuré pour afficher également des images prises antérieurement par la caméra,
- le dispositif de détection de fuites comporte au moins un filtre agencé entre le premier et le deuxième orifice de la canalisation de circulation de gaz de la sonde de reniflage,
- l'organe de contrôle porté par le gant comporte un moyen d'activation configuré pour piloter l'ouverture d'une vanne permettant le passage d'un flux de gaz traceur dans la canalisation de circulation de gaz de la soufflette d'aspersion.

L'invention a aussi pour objet un module de détection de fuites comprenant un détecteur de fuites et/ou une source de gaz traceur caractérisé en ce qu'il comporte un dispositif de détection de fuites tel que décrit précédemment.

Selon une ou plusieurs caractéristiques du module de détection de fuites, prise seule ou en combinaison :
- le détecteur de fuites comporte une unité de contrôle configurée pour communiquer avec un organe de contrôle porté par le gant du dispositif de détection de fuites,
- le module de détection de fuites comporte une vanne permettant le passage d'un flux de gaz traceur dans la canalisation de circulation de gaz de la soufflette d'aspersion, l'ouverture de la vanne étant pilotable par un moyen d'activation de l'organe de contrôle porté par le gant.

D'autres caractéristiques et avantages de l'invention ressortiront de la description suivante, donnée à titre d'exemple, sans caractère limitatif, en regard des dessins annexés sur lesquels:
- la figure 1 montre une vue schématique de la main d'un utilisateur portant un dispositif de détection de fuites,
- la figure 2 montre une vue schématique d'un exemple d'un dispositif de détection de fuites relié à un détecteur de fuites pour tester l'étanchéité d'un objet à tester en mode reniflage,
- la figure 3 montre un exemple d'affichage d'un système de vision déporté,
- la figure 4 montre une vue schématique d'un détail du dispositif de détection de fuites de la figure 2, et
- la figure 5 montre une vue schématique d'un exemple du dispositif de détection de fuites relié à une source de gaz traceur pour tester l'étanchéité d'un objet à tester en mode d'aspersion.

Sur ces figures, les éléments identiques portent les mêmes numéros de référence. Les réalisations suivantes sont des exemples. Bien que la description se réfère à un ou plusieurs modes de réalisation, ceci ne signifie pas nécessairement que chaque référence concerne le même mode de réalisation, ou que les caractéristiques s'appliquent seulement à un seul mode de réalisation. De simples caractéristiques de différents modes de réalisation peuvent également être combinées pour fournir d'autres réalisations.

Dans la suite de la description, on utilisera les termes « amont » et « aval » en référence à la direction d'écoulement des gaz.

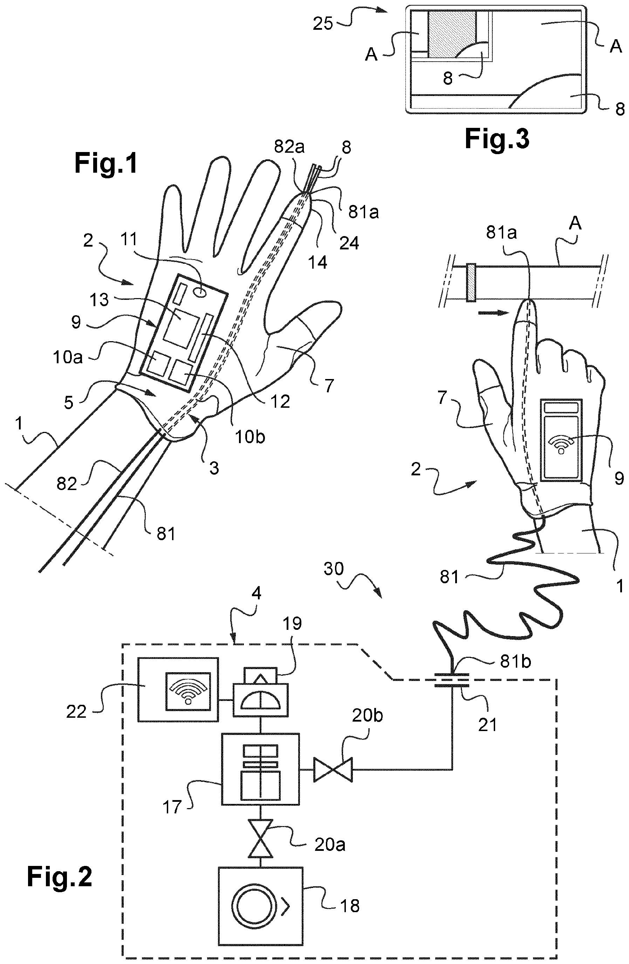

La figure 1 montre une vue schématique d'une main d'un utilisateur 1 portant un dispositif de détection de fuites 2 configuré pour contrôler l'étanchéité d'un objet à tester par gaz traceur.

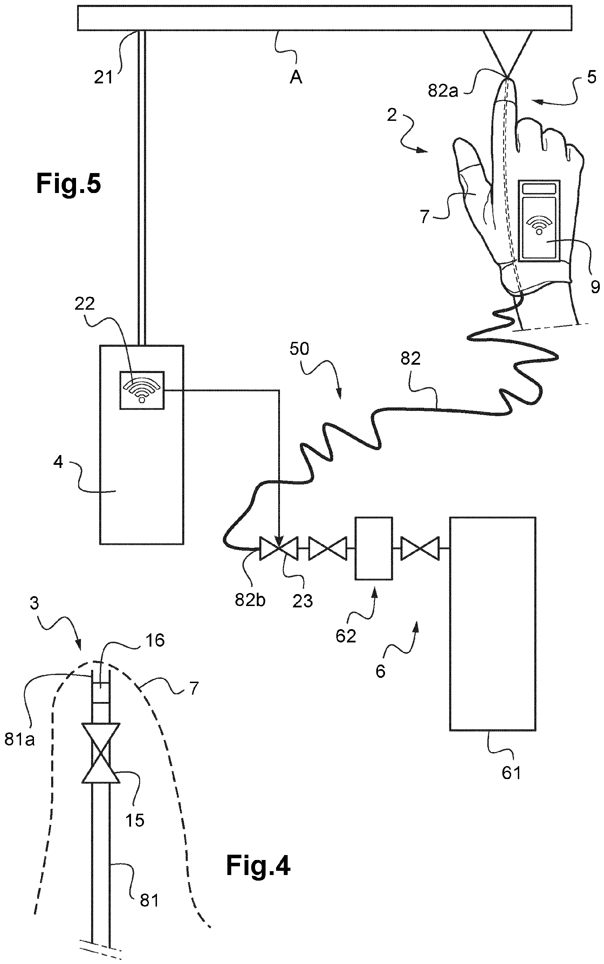

Le dispositif de détection de fuites 2 comprend une sonde de reniflage 3 destinée à être reliée à un détecteur de fuites 4 (figure 2) et/ou une soufflette d'aspersion 5 destinée à être reliée à une source de gaz traceur 6 (figure 5).

Sur l'exemple de la figure 1, le dispositif de détection de fuites 2 comprend une sonde de reniflage 3 et une soufflette d'aspersion 5, l'utilisateur pouvant choisir l'emploi de l'une ou l'autre pour tester un objet soit en mode reniflage, soit en mode d'aspersion. Le dispositif de détection de fuites 2 peut aussi ne comporter qu'une sonde de reniflage 3 (figure 2) ou qu'une soufflette d'aspersion 5 (figure 5).

On utilise généralement l'hélium ou l'hydrogène comme gaz traceur car ces gaz traversent les petites fuites plus aisément que les autres gaz, du fait de la petite taille de leur molécule et de leur grande vitesse de déplacement.

Le dispositif de détection de fuites 2 comporte également un gant 7 auquel la sonde de reniflage 3 et/ou la soufflette d'aspersion 5 est fixée (figure 1).

Le gant 7 peut être en matière textile, tel qu'en tissu et/ou en matériau élastique. Il épouse la forme de la main de l'utilisateur 1 en recouvrant de préférence les doigts et éventuellement le poignet pour assurer un meilleur maintien.

La sonde de reniflage 3 et/ou la soufflette d'aspersion 5 comporte au moins une canalisation de circulation de gaz 81, 82 comprenant un premier orifice 81a, 82a pour l'entrée ou la sortie de gaz et un deuxième orifice 81b, 82b destiné à être relié à un détecteur de fuites 4 ou à une source de gaz traceur 6.

La canalisation de circulation de gaz 81, 82 traverse le gant 7 en étant intégrée dans le gant 7 pour être protégée de l'environnement extérieur et pour éviter de s'accrocher lors des déplacements de l'utilisateur. Elle est par exemple cousue ou thermocollée au matériau textile du gant 7 entre deux bandes de tissu.

La canalisation de circulation de gaz 81, 82 sort du gant 7, par exemple au niveau du poignet, pour être reliée au détecteur de fuites 4 et/ou à la source de gaz traceur 6 par un ou plusieurs éléments de canalisation. A l'extérieur du gant 7, la canalisation de gaz 81, 82 peut ainsi présenter une longueur de plusieurs mètres.

Au niveau du gant 7, la canalisation de circulation de gaz 81, 82 s'étend par exemple le long du dos de la main ainsi que le long d'une partie 7a du gant 7 couvrant un doigt de l'utilisateur 1, tel que le long de l'index. La canalisation de circulation de gaz 81, 82 débouche par exemple du gant 7 au niveau de l'extrémité de ladite partie du gant 7 correspondant au bout du doigt de l'utilisateur 1 (figures 1, 2, 4 et 5). En particulier, une canalisation de circulation de gaz 81, 82 intégrée dans la partie dorsale du gant 7 permet de limiter les risques de déformation de celle-ci par fermeture du poing.

On prévoit en outre que la canalisation de circulation de gaz 81, 82 soit flexible, pour permettre les mouvements des doigts de la main. La canalisation 81, 82 est par exemple en matériau plastique.

Le dispositif de détection de fuites 2 peut en outre comporter un petit embout amovible 8, par exemple en forme tubulaire rigide, coopérant avec un moyen complémentaire porté par le gant 7, par exemple par vissage. Le moyen complémentaire peut être agencé au bout de la partie du gant 7 couvrant le doigt de l'utilisateur 1. Il est ainsi possible de prolonger la canalisation de circulation de gaz 81, 82 au niveau du premier orifice 81a, 82a pour faciliter l'accès de la sonde de reniflage 3 et/ou de la soufflette d'aspersion 5 dans des zones de recherches de petites dimensions, inférieures aux dimensions du doigt.

La sonde de reniflage 3 et/ou la soufflette d'aspersion 5 portée par le gant 7 permet ainsi à l'utilisateur 1 une manipulation plus intuitive et plus ergonomique. Il peut ainsi accéder plus facilement aux zones de l'objet à tester peu visibles, telles que des zones arrières ou sombres, avec la dextérité que lui procure l'adresse de sa main et avec un repérage spatial facilité par le contact manuel de l'objet à tester. De plus, le port de la sonde de reniflage 3 et/ou de la soufflette d'aspersion 5 par le gant 7 n'immobilise plus la main de l'utilisateur qui peut l'employer à d'autres fins. On évite en outre les douleurs aux mains liées à une prise prolongée des renifleurs/pistolets de l'art antérieur.

Le dispositif de détection de fuites 2 peut également comporter un organe de contrôle 9 configuré pour communiquer avec une unité de contrôle 22 du détecteur de fuites 4.

L'organe de contrôle 9 est par exemple fixé à la partie du gant 7 destinée à couvrir le dos de la main.

L'organe de contrôle 9 peut comporter au moins un moyen d'activation 10a, 10b configuré pour commander au moins un paramètre du détecteur de fuites 4 tel qu'une mesure de la concentration en gaz traceur représentative du taux de fuites de l'objet à tester A et/ou pour commander une réinitialisation du bruit de fond.

La mesure de la concentration en gaz traceur est réalisée au moyen d'un analyseur de gaz du détecteur de fuites 4 qui analyse les gaz prélevés par un dispositif de pompage du détecteur 4.

La réinitialisation du bruit de fond permet d'attribuer une valeur nulle à une mesure de la concentration en gaz traceur. Cela permet de déceler plus facilement la présence d'une fuite lorsque le niveau du bruit de fond est élevé sans attendre une baisse du niveau de gaz traceur et sans ventiler l'atmosphère.

Le moyen d'activation comporte par exemple un pavé tactile 10a, 10b, comprenant un capteur résistif ou capacitif, configuré pour détecter un appui d'un doigt de l'utilisateur 1. Ainsi, un appui sur un premier pavé tactile 10a permet de commander une mesure de la concentration en gaz traceur par le détecteur de fuites 4 et un appui sur un deuxième pavé tactile 10b permet de commander une réinitialisation du bruit de fond.

Les fonctions de la télécommande du détecteur de fuites 4 peuvent ainsi être agencées au niveau du gant 7, ce qui évite à l'utilisateur 1 de porter une télécommande et l'immobilisation de sa main. En outre, l'activation de l'organe de contrôle 9 par l'autre main est aisée.

D'autres moyens d'activation de l'organe de contrôle 9 peuvent être prévus.

Par exemple le moyen d'activation peut comporter un capteur de mouvement porté par le gant 7, configuré pour détecter un mouvement d'un doigt de la main de l'utilisateur 1 portant le gant 7. Selon un autre exemple, le moyen d'activation peut comporter des jauges de contraintes agencées sur deux parties du gant 7 couvrant les doigts de l'utilisateur 1 pour détecter un contact entre deux parties du gant 7. De la sorte, les mouvements des doigts peuvent suffire à l'utilisateur 1 pour commander un paramètre du détecteur de fuites 4, tel qu'une mesure de la concentration en gaz traceur ou une réinitialisation du bruit de fond.

L'organe de contrôle 9 peut aussi comporter des moyens d'activation configurés pour commander d'autres paramètres du détecteur de fuites 4, tels que le réglage du niveau sonore représentatif du taux de fuites mesuré par le détecteur de fuites 4. En effet, le détecteur de fuites 4 ou le dispositif de détection de fuites 2 peut comporter un dispositif d'émission sonore, tel qu'un bipper ou un hautparleur, dont la fréquence augmente avec l'augmentation du signal de mesure représentatif du taux de fuites. Le réglage du niveau sonore du dispositif d'émission sonore, à distance dans le détecteur de fuites 4 ou intégré dans le gant 7, peut permettre à l'utilisateur 1 éloigné du détecteur de fuites 4 ou dans un environnement bruyant, d'augmenter le son du dispositif d'émission sonore sans se déplacer.

Le dispositif de détection de fuites 2 peut aussi comporter un dispositif d'affichage porté par le gant 7, configuré pour afficher un signal de mesure du détecteur de fuites 4 et/ou pour donner une information de l'état du détecteur de fuites 4.

Le dispositif d'affichage communique avec l'unité de contrôle 22 du détecteur de fuites 4 pour récupérer les données de mesure de l'analyseur de gaz du détecteur 4.

Le dispositif d'affichage est par exemple fixé à une partie du gant 7 destinée à couvrir le dos de la main, par exemple à côté de l'organe de contrôle 9.

Le dispositif d'affichage peut comporter un premier indicateur visuel 11, tel qu'un voyant bicolore, pour donner une information de l'état du détecteur, à savoir si le détecteur de fuites 4 est en train de réaliser une mesure. L'information de l'état du détecteur de fuites 4 permet par exemple à l'utilisateur ne pouvant pas visualiser directement le détecteur de fuites 4, de s'assurer qu'il est bien en train de faire une mesure pour conclure à l'absence de fuites en présence d'un signal de mesure faible ou nul.

Le dispositif d'affichage peut comporter un deuxième indicateur visuel 12 pour afficher un signal de mesure de la concentration en gaz traceur envoyé par le détecteur de fuites 4.

Le deuxième indicateur visuel 12 est par exemple un écran ou un bargraphe, affichant sous forme de barres graphiques d'intensité ou sous forme chiffrée ou exprimée en pourcentage ou sous forme de pictogrammes particuliers, des informations sur l'intensité de la concentration en gaz traceur, représentative du taux de fuite de l'objet à tester sur une ou plusieurs décades.

Le deuxième indicateur visuel 12 peut également être configuré pour changer de couleur lorsque le signal de mesure franchit au moins un seuil de détection.

Le dispositif d'affichage peut comporter un écran 13 configuré pour afficher une évolution temporelle du signal de mesure de la concentration en gaz traceur. Cela peut permettre à l'utilisateur 1 de comparer plusieurs pics entre eux dans le signal de mesure et ainsi par exemple de déterminer plus facilement la localisation de la fuite ayant le taux de fuites le plus important.

On peut aussi prévoir que le dispositif d'affichage et les moyens d'activation 10a, 10b de l'organe de contrôle 9 soient formés par une seule et même interface, telle qu'un écran tactile.

Le dispositif de détection de fuites 2 peut en outre comporter un actionneur vibratoire porté par le gant 7, agencé par exemple au niveau de l'écran tactile, pour faire vibrer le gant 7 par exemple lorsque le détecteur de fuites 4 lance une mesure ou lorsque le signal de mesure franchit au moins un seuil de détection.

L'organe de contrôle 9 et/ou le dispositif d'affichage 11, 12, 13 peuvent comporter des moyens de communication sans fils pour communiquer avec une unité de contrôle 22 du détecteur de fuites 4. Les moyens de communication sans fils sont par exemple de type WIFI, Bluetooth ou autre. On limite ainsi les liaisons filaires potentiellement gênantes entre le dispositif de détection de fuites 2 et l'utilisateur 1.

Le dispositif de détection de fuites 2 peut aussi comporter un dispositif d'éclairage 14 porté par le gant 7, comprenant une source lumineuse telle qu'une diode électroluminescente (DEL ou LED pour « Light-Emitting Diode » en anglais), un commutateur pour allumer/éteindre la source lumineuse et une pile d'alimentation de la source lumineuse ou un câble d'alimentation à une source d'alimentation déportée.

La source lumineuse du dispositif d'éclairage 14 est par exemple agencée au niveau de l'extrémité de la partie du gant 7 couvrant le doigt de l'utilisateur 1, c'est-à-dire au bout du doigt et à proximité du premier orifice 81a de la canalisation de circulation de gaz 81. On peut ainsi éclairer facilement et avec précision la zone de recherche autour de l'objet à tester sans que la main de l'utilisateur 1 ne soit immobilisée pour la manipulation d'une lampe de poche.

On peut aussi prévoir un code couleur pour la lumière d'éclairage produite par le dispositif d'éclairage 14, défini en fonction du franchissement par le signal de mesure d'un ou de plusieurs seuils de détection. L'utilisateur 1 peut ainsi facilement et rapidement visualiser le taux de fuites mesuré sans avoir besoin de libérer une main pour saisir une télécommande.

Le dispositif de détection de fuites 2 peut aussi comporter une caméra 24 portée par le gant 7, par exemple intégrée ou fixée au gant 7 au bout de l'index, à côté des orifices 81a, 82a, et un système de vision déporté 25 configuré pour afficher les images prises par la caméra 24 (figure 3).

La caméra 24 permet de faciliter la visualisation par l'utilisateur lors d'une détection de fuites dans des zones peu accessibles où il n'y a pas de vision directe possible. Les images vidéos sont affichées sur le système de vision déporté 25 directement visible par l'utilisateur tel qu'un écran déporté par rapport au gant 7 et au détecteur de fuites 4 ou tel que des lunettes à réalité augmentée.

Les images sont par exemple diffusées par défaut en temps réel et permettent de visualiser la localisation du gant 7, c'est-à-dire des orifices 81a, 82a par rapport à l'objet à tester A.

Le système de vision déporté 25 peut également être configuré pour afficher des images prises antérieurement par la caméra 24. Cet affichage additionnel peut par exemple être localisé dans un coin de l'écran du système de vision déporté 25 affichant par ailleurs les images diffusées en temps réel (figure 3).

En effet, en cas de fuite détectée en mode reniflage, un délai peut exister entre le moment où un mélange gazeux s'échappant de la fuite est aspiré et le moment de la détection proprement dite par le détecteur de fuites 4. En affichant les images prises antérieurement, on facilite la localisation de la fuite lorsque le gant 7 se déplace : on évite qu'au moment de la détection de la fuite, le gant 7 se soit déplacé d'une certaine distance et que l'image vue par l'utilisateur ne corresponde plus à la localisation exacte de la fuite.

Les images prises antérieurement peuvent être affichées avec un décalage temporel prédéterminé. Ces images antérieures peuvent défiler en continu ou être remplacées par exemple sur commande de l'unité de contrôle 22, lorsque la concentration en gaz traceur dépasse un seuil prédéterminé ou un niveau précédent de concentration en gaz traceur.

Le traitement d'image permet, pour une sonde de reniflage 3 donnée (conductance donnée), d'afficher l'image prise par la caméra 24 avec un décalage temporel prédéterminé, correspondant à la position « réelle » de la fuite. Ce délai est déterminé par la conductance prédéfinie 15 de la canalisation de circulation des gaz 81 de la sonde de reniflage 3. Typiquement, et suivant la géométrie de la canalisation de circulation des gaz 81, ce délai peut aller de quelques dixièmes de secondes à quelques dizaines de secondes. Il est toutefois parfaitement défini pour une géométrie de canalisation donnée.

Les images prises antérieurement affichées sur le système de vision déporté 25 peuvent aussi correspondre à des arrêts sur image saisis pour des pics de concentration en gaz traceur sur commande de l'unité de contrôle 22. Les images peuvent par exemple être renouvelées lorsque la concentration en gaz traceur dépasse un seuil prédéterminé ou dépasse un niveau précédent de concentration en gaz traceur.

Les figures 2 et 4 montrent plus précisément un exemple de réalisation d'un module de détection de fuites 30 comprenant un détecteur de fuites 4 et un dispositif de détection de fuites 2 raccordé au détecteur de fuites 4 par une canalisation de circulation de gaz 81.

Comme on peut mieux le voir sur la figure 4, le dispositif de détection de fuites 2 comporte une conductance prédéfinie 15 agencée entre le premier et le deuxième orifice 81a, 81b de la canalisation de circulation de gaz 81 de la sonde de reniflage 3. La conductance prédéfinie 15 est configurée pour limiter le flux de gaz aspiré par le dispositif de pompage du détecteur de fuites 4. La conductance prédéfinie 15 est par exemple réalisée par un capillaire, un gicleur tel qu'un rubis percé, ou une membrane poreuse ou une aiguille.

Le dispositif de détection de fuites 2 comporte également au moins un filtre 16 monté dans la canalisation de circulation de gaz 81, en série de la conductance prédéfinie 15, par exemple en amont de cette dernière.

Le filtre 16 filtre les poussières pouvant provenir de l'atmosphère extérieure, permettant ainsi d'éviter à la canalisation de circulation de gaz 81 de se colmater. On peut prévoit plusieurs filtres en série, en amont de la conductance prédéfinie 15, tels qu'un premier filtre en métal fritté pour les poussières comprises entre 10 et 20 µm et un deuxième filtre à base de fibres de feutre pour les poussières plus fines par exemple comprises entre 5 et 10 µm.

On prévoit en outre d'approcher le filtre 16 au plus près du premier orifice 81a de la canalisation 81 de manière à éviter la pollution de la canalisation 81.

La conductance prédéfinie 15 peut être agencée dans la portion de la canalisation de circulation de gaz 81 qui est intégrée dans le gant 7 ou peut être éloignée du filtre 16, par exemple en étant agencée au niveau du bras de l'utilisateur.

En revenant à la figure 2, on voit que le détecteur de fuites 4 comporte un analyseur de gaz 19, tel qu'un spectromètre de masse, et un dispositif de pompage.

Selon un exemple de réalisation, le détecteur de fuites 4 comporte une unité de contrôle 22, tel qu'un microcontrôleur ou ordinateur, configurée pour communiquer avec l'organe de contrôle 9 porté par le gant 7.

L'unité de contrôle 22 est configuré pour lancer une mesure de la concentration en gaz traceur par l'analyseur de gaz 19 et/ou pour commander une réinitialisation du bruit de fond, à la réception d'un signal envoyé par l'organe de contrôle 9 du dispositif de détection de fuites 2.

Le dispositif de pompage comporte par exemple une pompe secondaire 17, telle qu'une pompe turbomoléculaire, et une pompe primaire 18, montées en série. La pompe primaire 18 est par exemple une pompe à membrane, qui aspire les gaz et les refoule à la pression atmosphérique ayant des vitesses de pompage de l'ordre de 0,25 m³/h à 4,3 m³/h. L'analyseur de gaz 19 est raccordé à l'aspiration de la pompe secondaire 17 dont le refoulement est raccordé à l'aspiration de la pompe primaire 18, via une première vanne d'isolation 20a. L'entrée 21 du détecteur de fuites 4, agencée en amont du dispositif de pompage via une deuxième vanne d'isolation 20b, est raccordée au deuxième orifice 81b de la canalisation de circulation de gaz 81 du dispositif de détection de fuites 2, directement ou par l'intermédiaire de canalisations flexibles intermédiaires.

En fonctionnement, l'objet à tester A est rempli en gaz traceur, généralement pressurisé, et on recherche la présence éventuelle du gaz traceur en déplaçant le doigt autour de l'objet à tester A. La sonde de reniflage 3 reliée au détecteur de fuites 4 et portée par le gant 7 aspire les gaz environnants par le premier orifice 81a. Une partie des gaz ainsi prélevés, contenant éventuellement le gaz traceur révélateur d'une fuite, est alors analysée par l'analyseur de gaz 19 qui fournit un signal de mesure de la concentration en gaz traceur.

La figure 5 montre un exemple de réalisation d'un dispositif de détection de fuites 2 raccordé à une source de gaz traceur 6 par une canalisation de circulation de gaz 82.

La source de gaz traceur 6 comporte par exemple une bouteille de gaz traceur 61 pressurisée et un détendeur 62 raccordé à la bouteille de gaz traceur 61.

Le module de détection de fuites 50 comporte en outre une vanne 23, telle qu'une électrovanne, permettant le passage d'un flux de gaz traceur dans la canalisation de circulation de gaz 82 de la soufflette d'aspersion 5. La vanne 23 est par exemple agencée à la sortie de la source de gaz traceur 6, interposée entre la sortie du détendeur 62 et le deuxième orifice 82b de la canalisation de circulation de gaz 82 de la soufflette d'aspersion 5.

L'ouverture de la vanne 23 peut être pilotée par un moyen d'activation de l'organe de contrôle 9 porté par le gant 7.

Ainsi, le pilotage en ouverture de la vanne 23 peut être réalisée par des moyens de communication sans fils, par des moyens d'activation agencés sur le gant 7 par exemple de type capteur tactile tel qu'un écran tactile, un pavé tactile ou des jauges de contraintes ou tel qu'un capteur de mouvement. De la sorte, les mouvements des doigts peuvent suffire à l'utilisateur 1 pour commander la mise en route du soufflage de gaz traceur par la soufflette d'aspersion 5.

La source de gaz 6 et le détecteur de fuites 4 peuvent en outre être portés par un châssis commun, pour faciliter le déplacement du module de détection 30, 50 et pour faciliter la connexion de la vanne 23 à l'unité de contrôle 22 du détecteur de fuites 4.

En fonctionnement, l'objet à tester A est raccordé à un détecteur de fuites 4, par exemple tel que celui décrit dans l'exemple de la figure 2, et éventuellement à un dispositif de pompage auxiliaire (non représenté).

On recherche la présence éventuelle du gaz traceur, en déplaçant le doigt, et ainsi le premier orifice 82a de la soufflette d'aspersion 5, autour de l'objet à tester A pour l'asperger de gaz traceur. L'entrée 21 du détecteur de fuites 4 prélève une partie des gaz contenus dans l'objet à tester A. Une partie des gaz ainsi prélevés, contenant éventuellement le gaz traceur révélateur d'une fuite, est alors analysée par l'analyseur de gaz 19 qui fournit un signal de mesure de la concentration en gaz traceur.

## Revendications

1. Dispositif de détection de fuites (2) configuré pour contrôler l'étanchéité d'un objet à tester par gaz traceur, ledit dispositif de détection de fuites (2) comprenant une sonde de reniflage (3) destinée à être reliée à un détecteur de fuites (4), **caractérisé en ce qu'**il comporte un gant (7) auquel la sonde de reniflage (3) est fixée, la sonde de reniflage (3) comportant une canalisation de circulation des gaz (81) comprenant un premier orifice (81a) pour l'entrée des gaz et un deuxième orifice (81b) destiné à être relié à un détecteur de fuites (4), le dispositif de détection de fuites (2) comportant une conductance prédéfinie (15) agencée entre le premier et le deuxième orifice (81a, 81b) de la canalisation de circulation de gaz (81) de la sonde de reniflage (3), la conductance prédéfinie (15) étant configurée pour limiter le flux de gaz destiné à être aspiré par un dispositif de pompage du détecteur de fuites (4).

2. Dispositif de détection de fuites (2) configuré pour contrôler l'étanchéité d'un objet à tester par gaz traceur, ledit dispositif de détection de fuites (2) comprenant une soufflette d'aspersion (5) destinée à être reliée à une source de gaz traceur (6), **caractérisé en ce qu'**il comporte un gant (7) auquel la soufflette d'aspersion (5) est fixée, la soufflette d'aspersion (5) comportant une canalisation de circulation des gaz (82) comprenant un premier orifice (82a) pour la sortie des gaz et un deuxième orifice (82b) destiné à être relié à une source de gaz traceur (6).

3. Dispositif de détection de fuites (2) selon l'une des revendications précédentes, **caractérisé en ce que** la canalisation de circulation de gaz (81, 82) est flexible.

4. Dispositif de détection de fuites (2) selon l'une des revendications précédentes, **caractérisé en ce que** la canalisation de circulation de gaz (81, 82) traverse le gant (7) en étant intégrée dans le gant (7).

5. Dispositif de détection de fuites (2) selon l'une des revendications précédentes, **caractérisé en ce que** la canalisation de circulation de gaz (81, 82) s'étend le long d'une partie du gant (7) destinée à couvrir un doigt de l'utilisateur (1), en particulier l'index, et débouche au niveau de l'extrémité de ladite partie du gant (7) correspondant au bout du doigt.

6. Dispositif de détection de fuites (2) selon l'une des revendications précédentes, **caractérisé en ce qu'**il comporte un organe de contrôle (9) porté par le gant (7) et configuré pour communiquer avec une unité de contrôle (22) du détecteur de fuites (4).

7. Dispositif de détection de fuites (2) selon la revendication précédente, **caractérisé en ce que** l'organe de contrôle (9) comporte au moins un moyen d'activation (10a, 10b) configuré pour commander le lancement d'une mesure de la concentration en gaz traceur et/ou d'une réinitialisation du bruit de fond du détecteur de fuites (4).

8. Dispositif de détection de fuites (2) selon l'une des revendications précédentes, **caractérisé en ce qu'**il comporte un dispositif d'affichage (11, 12, 13) porté par le gant (7), configuré pour afficher un signal de mesure du détecteur de fuites (4) et/ou pour donner une information de l'état du détecteur de fuites (4).

9. Dispositif de détection de fuites (2) selon la revendication précédente, **caractérisé en ce que** le dispositif d'affichage comporte un écran (13) configuré pour afficher une évolution de la concentration en gaz traceur au cours du temps mesuré par le détecteur de fuites (4).

10. Dispositif de détection de fuites (2) selon l'une des revendications 6 à 9, **caractérisé en ce que** l'organe de contrôle (9) et/ou le dispositif d'affichage (11, 12, 13) comportent des moyens de communication sans fils pour communiquer avec une unité de contrôle (22) du détecteur de fuites (4).

11. Dispositif de détection de fuites (2) selon l'une des revendications précédentes, **caractérisé en ce qu'**il comporte un dispositif d'éclairage (14) porté par le gant (7).

12. Dispositif de détection de fuites (2) selon l'une des revendications précédentes, **caractérisé en ce qu'**il comporte une caméra (24) portée par le gant (7) et un système de vision déporté (25) configuré pour afficher les images prises par la caméra (24).

13. Dispositif de détection de fuites (2) selon la revendication précédente, **caractérisé en ce que** le système de vision déporté (25) est configuré pour afficher également des images prises antérieurement par la caméra (24).

14. Dispositif de détection de fuites (2) selon l'une des revendications précédentes, prise avec la revendication 1, **caractérisé en ce qu'**il comporte au moins un filtre (16) agencé entre le premier et le deuxième orifice (81a, 81b) de la canalisation de circulation de gaz (81) de la sonde de reniflage (3).

15. Dispositif de détection de fuites (2) selon l'une des revendications 6 à 14, prise ensemble avec la revendication 2, **caractérisé en ce que** l'organe de contrôle (9) porté par le gant (7) comporte un moyen d'activation configuré pour piloter l'ouverture d'une vanne (23) permettant le passage d'un flux de gaz traceur dans la canalisation de circulation de gaz (82) de la soufflette d'aspersion (5).

16. Module de détection de fuites (30, 50) comprenant un détecteur de fuites (4) et/ou une source de gaz traceur (6) **caractérisé en ce qu'**il comporte un dispositif de détection de fuites (2) selon l'une des revendications précédentes.

17. Module de détection de fuites (30, 50) selon la revendication précédente, **caractérisé en ce que** le détecteur de fuites (4) comporte une unité de contrôle (22) configurée pour communiquer avec un organe de contrôle (9) porté par le gant (7) du dispositif de détection de fuites (2).

18. Module de détection de fuites (30, 50) selon l'une des revendications 16 ou 17, prise ensemble avec la revendication 15, **caractérisé en ce qu'**il comporte une vanne (23) permettant le passage d'un flux de gaz traceur dans la canalisation de circulation de gaz (82) de la soufflette d'aspersion (5), l'ouverture de la vanne (23) étant pilotable par un moyen d'activation de l'organe de contrôle (9) porté par le gant (7).

## Patentansprüche

1. Leckerkennungsvorrichtung (2), die konfiguriert ist, die Dichtheit eines zu testenden Gegenstands durch Spürgas zu überwachen, wobei die Leckerkennungsvorrichtung (2) eine Schnüffelsonde (3) enthält, die dazu bestimmt ist, mit einem Leckdetektor (4) verbunden zu werden, **dadurch gekennzeichnet, dass** sie einen Handschuh (7) aufweist, an dem die Schnüffelsonde (3) befestigt ist, wobei die Schnüffelsonde (3) eine Gasdurchflussleitung (81) aufweist, die eine erste Öffnung (81a) für den Eintritt der Gase und eine zweite Öffnung (81b) enthält, die dazu bestimmt ist, mit einem Leckdetektor (4) verbunden zu werden, wobei die Leckerkennungsvorrichtung (2) eine vordefinierte Konduktanz (15) aufweist, die zwischen der ersten und der zweiten Öffnung (81a, 81b) der Gasdurchflussleitung (81) der Schnüffelsonde (3) angeordnet ist, wobei die vordefinierte Konduktanz (15) konfiguriert ist, den Gasstrom zu begrenzen, der dazu bestimmt ist, von einer Pumpvorrichtung des Leckdetektors (4) angesaugt zu werden.

2. Leckerkennungsvorrichtung (2), die konfiguriert ist, die Dichtheit eines zu testenden Gegenstands durch Spürgas zu überwachen, wobei die Leckerkennungsvorrichtung (2) einen Sprühzerstäuber (5) enthält, der dazu bestimmt ist, mit einer Spürgasquelle (6) verbunden zu werden, **dadurch gekennzeichnet, dass** sie einen Handschuh (7) aufweist, an dem der Sprühzerstäuber (5) befestigt ist, wobei der Sprühzerstäuber (5) eine Gasdurchflussleitung (82) aufweist, die eine erste Öffnung (82a) für den Austritt der Gase und eine zweite Öffnung (82b) enthält, die dazu bestimmt ist, mit einer Spürgasquelle (6) verbunden zu werden.

3. Leckerkennungsvorrichtung (2) nach einem der vorhergehenden Ansprüche, **dadurch gekennzeichnet, dass** die Gasdurchflussleitung (81, 82) biegsam ist.

4. Leckerkennungsvorrichtung (2) nach einem der vorhergehenden Ansprüche, **dadurch gekennzeichnet, dass** die Gasdurchflussleitung (81, 82) den Handschuh (7) durchquert, indem sie in den Handschuh (7) integriert ist.

5. Leckerkennungsvorrichtung (2) nach einem der vorhergehenden Ansprüche, **dadurch gekennzeichnet, dass** die Gasdurchflussleitung (81, 82) sich entlang eines Teils des Handschuhs (7) erstreckt, der dazu bestimmt ist, einen Finger des Benutzers (1), insbesondere den Zeigefinger, zu bedecken, und im Bereich des Endes des Teils des Handschuhs (7) mündet, der der Fingerspitze entspricht.

6. Leckerkennungsvorrichtung (2) nach einem der vorhergehenden Ansprüche, **dadurch gekennzeichnet, dass** sie ein Überwachungsorgan (9) aufweist, das vom Handschuh (7) getragen wird und konfiguriert ist, mit einer Überwachungseinheit (22) des Leckdetektors (4) zu kommunizieren.

7. Leckerkennungsvorrichtung (2) nach dem vorhergehenden Anspruch, **dadurch gekennzeichnet, dass** das Überwachungsorgan (9) mindestens eine Aktivierungseinrichtung (10a, 10b) aufweist, die konfiguriert ist, den Start einer Messung der Spürgaskonzentration und/oder einer Rücksetzung des Hintergrundrauschens des Leckdetektors (4) zu steuern.

8. Leckerkennungsvorrichtung (2) nach einem der vorhergehenden Ansprüche, **dadurch gekennzeichnet, dass** sie eine vom Handschuh (7) getragene Anzeigevorrichtung (11, 12, 13) aufweist, die konfiguriert ist, ein Messsignal des Leckdetektors (4) anzuzeigen und/oder eine Information über den Zustand des Leckdetektors (4) anzugeben.

9. Leckerkennungsvorrichtung (2) nach dem vorhergehenden Anspruch, **dadurch gekennzeichnet, dass** die Anzeigevorrichtung einen Bildschirm (13) aufweist, der konfiguriert ist, eine Entwicklung der Spürgaskonzentration während der vom Leckdetektor (4) gemessenen Zeit anzuzeigen.

10. Leckerkennungsvorrichtung (2) nach einem der Ansprüche 6 bis 9, **dadurch gekennzeichnet, dass** das Überwachungsorgan (9) und/oder die Anzeigevorrichtung (11, 12, 13) Einrichtungen zur drahtlosen Kommunikation aufweisen, um mit einer Überwachungseinheit (22) des Leckdetektors (4) zu kommunizieren.

11. Leckerkennungsvorrichtung (2) nach einem der vorhergehenden Ansprüche, **dadurch gekennzeichnet, dass** sie eine vom Handschuh (7) getragene Beleuchtungsvorrichtung (14) aufweist.

12. Leckerkennungsvorrichtung (2) nach einem der vorhergehenden Ansprüche, **dadurch gekennzeichnet, dass** sie eine vom Handschuh (7) getragene Kamera (24) und ein ausgelagertes Sichtsystem (25) aufweist, das konfiguriert ist, die von der Kamera (24) aufgenommenen Bilder anzuzeigen.

13. Leckerkennungsvorrichtung (2) nach dem vorhergehenden Anspruch, **dadurch gekennzeichnet, dass** das ausgelagerte Sichtsystem (25) konfiguriert ist, ebenfalls vorher von der Kamera (24) aufgenommene Bilder anzuzeigen.

14. Leckerkennungsvorrichtung (2) nach einem der vorhergehenden Ansprüche in Verbindung mit Anspruch 1, **dadurch gekennzeichnet, dass** sie mindestens einen Filter (16) aufweist, der zwischen der ersten und der zweiten Öffnung (81a, 81b) der Gasdurchflussleitung (81) der Schnüffelsonde (3) angeordnet ist.

15. Leckerkennungsvorrichtung (2) nach einem der Ansprüche 6 bis 14 in Verbindung mit Anspruch 2, **dadurch gekennzeichnet, dass** das vom Handschuh (7) getragene Überwachungsorgan (9) eine Aktivierungseinrichtung aufweist, die konfiguriert ist, die Öffnung eines Ventils (23) zu steuern, das den Durchgang eines Spürgasstroms in der Gasdurchflussleitung (82) des Sprühzerstäubers (5) erlaubt.

16. Leckerkennungsmodul (30, 50), das einen Leckdetektor (4) und/oder eine Spürgasquelle (6) aufweist, **dadurch gekennzeichnet, dass** es eine Leckerkennungsvorrichtung (2) nach einem der vorhergehenden Ansprüche aufweist.

17. Leckerkennungsmodul (30, 50) nach dem vorhergehenden Anspruch, **dadurch gekennzeichnet, dass** der Leckdetektor (4) eine Überwachungseinheit (22) aufweist, die konfiguriert ist, mit einem vom Handschuh (7) der Leckerkennungsvorrichtung (2) getragenen Überwachungsorgan (9) zu kommunizieren.

18. Leckerkennungsmodul (30, 50) nach einem der Ansprüche 16 oder 17 in Verbindung mit Anspruch 15, **dadurch gekennzeichnet, dass** es ein Ventil (23) aufweist, das den Durchgang eines Spürgasstroms in der Gasdurchflussleitung (82) des Sprühzerstäubers (5) erlaubt, wobei die Öffnung des Ventils (23) von einer Aktivierungseinrichtung des vom Handschuh (7) getragenen Überwachungsorgan (9) gesteuert werden kann.

## Claims

1. Device for the detection of leaks (2) configured to check the tightness of an object to be tested by tracer gas, said leak detection device (2) comprising a sniffing probe (3) intended to be connected to a leak detector (4), **characterized in that** it includes a glove (7) to which the sniffing probe (3) is attached, the sniffing probe (3) including a line for the circulation of the gases (81) comprising a first orifice (81a) for the inlet of the gases and a second orifice (81b) intended to be connected to a leak detector (4), the device for the detection of leaks (2) including a predefined conductance (15) arranged between the first and the second orifice (81a, 81b) of the gas circulation line (81) of the sniffing probe (3), the predefined conductance (15) being configured to limit the flow of gas intended to be drawn in via a pumping device of the leak detector (4).

2. Device for the detection of leaks (2) configured to check the tightness of an object to be tested by tracer gas, said leak detection device (2) comprising a spray blower (5) intended to be connected to a source of tracer gas (6), **characterized in that** it includes a glove (7) to which the spray blower (5) is attached, the spray blower (5) including a line for the circulation of the gases (82) comprising a first orifice (82a) for the outlet of the gases and a second orifice (82b) intended to be connected to a source of tracer gas (6).

3. Device for the detection of leaks (2) according to either of the preceding claims, **characterized in that** the gas circulation line (81, 82) is flexible.

4. Device for the detection of leaks (2) according to one of the preceding claims, **characterized in that** the gas circulation line (81, 82) passes through the glove (7) by being integrated into the glove (7) .

5. Device for the detection of leaks (2) according to one of the preceding claims, **characterized in that** the gas circulation line (81, 82) extends along a part of the glove (7) intended to cover a finger of the user (1), in particular the index finger, and leads to the level of the extremity of said part of the glove (7) corresponding to the tip of the finger.

6. Device for the detection of leaks (2) according to one of the preceding claims, **characterized in that** it includes a control device (9) carried by the glove (7) and configured to communicate with a control unit (22) of the leak detector (4).

7. Device for the detection of leaks (2) according to the preceding claim, **characterized in that** the control device (9) includes at least one means of activation (10a, 10b) configured to control the initiation of a measurement of the concentration of tracer gas and/or of a reinitialization of the background noise of the leak detector (4).

8. Device for the detection of leaks (2) according to one of the preceding claims, **characterized in that** it includes a display device (11, 12, 13) carried by the glove (7), configured to display a measurement signal from the leak detector (4) and/ or in order to provide information in respect of the state of the leak detector (4).

9. Device for the detection of leaks (2) according to the preceding claim, **characterized in that** the display device includes a screen (13) configured to display the evolution in the concentration of tracer gas over time measured by the leak detector (4) .

10. Device for the detection of leaks (2) according to one of Claims 6 to 9, **characterized in that** the control device (9) and/or the display device (11, 12, 13) include means of wireless communication in order to communicate with a control unit (22) of the leak detector (4).

11. Device for the detection of leaks (2) according to one of the preceding claims, **characterized in that** it includes an illumination device (14) carried by the glove (7).

12. Device for the detection of leaks (2) according to one of the preceding claims, **characterized in that** it includes a camera (24) carried by the glove (7) and a remote visualization system (25) configured to display the images taken by the camera (24).

13. Device for the detection of leaks (2) according to the preceding claim, **characterized in that** the remote visualization system (25) is also configured to display images taken earlier by the camera (24).

14. Device for the detection of leaks (2) according to one of the preceding claims, considered together with Claim 1, **characterized in that** it includes at least one filter (16) arranged between the first and the second orifice (81a, 81b) of the gas circulation line (81) of the sniffing probe (3).

15. Device for the detection of leaks (2) according to one of Claims 6 to 14, considered together with Claim 2, **characterized in that** the control device (9) carried by the glove (7) includes a means of activation configured to operate the opening of a valve (23) permitting the passage of a flow of tracer gas in the gas circulation line (82) of the spray blower (5).

16. Module for the detection of leaks (30, 50) comprising a leak detector (4) and/or a source of tracer gas (6), **characterized in that** it includes a leak detection device (2) according to one of the preceding claims.

17. Module for the detection of leaks (30, 50) according to the preceding claim, **characterized in that** the leak detector (4) includes a control unit (22) configured to communicate with a control device (9) carried by the glove (7) of the leak detection device (2).

18. Module for the detection of leaks (30, 50) according to either of Claims 16 and 17, considered together with Claim 15, **characterized in that** it includes a valve (23) permitting the passage of a flow of tracer gas in the gas circulation line (82) of the spray blower (5), the opening of the valve (23) being capable of operation via a means of activation of the control device (9) carried by the glove (7).
